# FASCICULE DE BREVET EUROPEEN

(11) **EP 1 164 956 B1**
(45) Date de publication et mention de la délivrance du brevet: **11.06.2008**
(21) Numéro de dépôt: 00931355.2
(22) Date de dépôt: 26.05.2000
(51) Int. Cl.: A61B 17/70

(54) **DISPOSITIF DE DISTRACTION POUR LES OS D'ENFANTS**
KNOCHENDISTRAKTIONSVORRICHTUNG FÜR KINDER
DISTRACTION DEVICE FOR THE BONES OF CHILDREN

(30) Priorité: 01.06.1999 FR 9907034
(43) Date de publication de la demande: 02.01.2002
(73) Titulaire: Fortin, Frédéric, 33600 Pessac (FR)
(72) Inventeur: Fortin, Frédéric, 33600 Pessac (FR)
(74) Mandataire: Molnia, David
(86) Numéro de dépôt international: PCT/FR2000/001427
(87) Numéro de publication internationale: WO 2000/072768

(56) Documents cités:
- DE-A- 2 437 752
- US-A- 4 386 603
- US-A- 4 611 582
- US-A- 4 658 809
- US-A- 4 931 055

## Description

### DOMAINE DE L'INVENTION

L'invention concerne un dispositif de mise en tension que l'on place entre les côtes d'un enfant dont la croissance n'est pas terminée et qui présente des déformations ou malformations notamment du tronc .Dans ce cas présent les chirurgiens spécialistes de ce type d'intervention l'appellent : dispositif de distraction . Il va permettre de corriger aussi bien les malformations congénitales que celles qui apparaîtraient en cours de croissance, sans interdire ni bloquer celle-ci ;c'est donc en particulier grâce aux moyens mis en jeu que cette invention saura résoudre le problème posé , jusqu'ici non résolu.

### ART ANTERIEUR

Les premiers traitements des scolioses étaient basés sur des mouvements de la colonne vertébrale. Y Cotrel perfectionne cette technique en mettant au point en 1982 un dispositif de traction et d'élongation du rachis. Cette traction du rachis était très douloureuse. Pour les cas les plus graves ,elle était suivie d'un platrage du thorax pour tenter de garder le mieux possible la correction effectuée. Le plus souvent le médecin prescrivait le port d'un corset qui maintenait plus ou moins le rachis. Pour les déformations les plus graves, les résultats étaient loin d'être satisfaisants .Le rachis ne se redressait pas suffisamment et le port du corset était astreignant. Malgré tous ces inconvénients cette méthode est encore utilisée pour le traitement des scolioses des jeunes enfants.

Il y a environ 30 ans Harrington a été le premier à implanter dans le dos d'un patient une tige avec deux crochets fixée par ses extrémités à l'ossature pour essayer de redresser la colonne. Le rachis étaient bien redressé mais 6 mois plus tard, une fois la greffe prise les vertèbres formaient un bloc osseux limitant la mobilité aux vertèbres non instrumentées.

La distance souvent importante entre les deux crochets faisait que la tige qui les reliait subissait de fortes contraintes , entraînant des ruptures de tiges et obligeait ainsi le chirurgien à intervenir. Une immobilisation de 6 mois par plâtre était souvent nécessaire. De plus la correction n'était effectuée que sur un seul plan. La balance sagittale (lordose lombaire et cyphose thoracique anatomiques) n'était pas respectée, il s'en suivait des problèmes en post opératoire pour le patient .

D'autres dispositifs ont été conçus pour permettre d'appliquer un écartement entre des os avec blocage.

Le document US -A-4611582 divulgue un dispositif de distraction comportant des moyens d'accrochages aux os, deux tiges montées sur un moyen de réglage central pourvu d'un petit trou d'engagement d'un petit outil destiné à régler la distance qui sépare les moyens d'accrochage aux os, et des moyens de blocage qui permettent de bloquer les deux tiges.

Le document DE-A-2437752 décrit un fixateur pour os externes longs dont les moyens d'accrochage sont des broches ou des vis et dont l'écartement est provoqué par une roue dentée qui fait déplacer deux tiges non identiques, massives, présentant un épaulement qui permet de pouvoir translater les moyens d'accrochage sur le même axe.

L'invention va être décrite dans trois modes de réalisations illustratifs et non limitatifs va permettre de résoudre ces problèmes auquels sont confrontés tous les enfants dont la croissance n'est pas terminée .

### DESCRIPTION

Le présent Dispositif de distraction se compose de dispositifs d'accrochage aux os adaptables par exemple aux côtes, et de dispositifs de réglage de l'intervalle situé entre deux dispositifs d'accrochage.

Les dispositifs d'accrochage sont indépendants de la distance d'origine des os à écarter: par exemple on peut accrocher une extrémité du dispositif d'accrochage d'une côte donnée à une autre cote qui n'est pas forcément la plus proche.

Les moyens de réglage vont permettre à la fois de régler et de corriger la déformation qui va évoluer avec la croissance en répercutant ce mouvement sur l'ensemble du dispositif de distraction, dont on peut facilement modifier périodiquement le réglage.

Nous décrirons cette invention dans trois modes de réalisations privilégiées qui permettent de l'adapter au cas par cas au problème posé en illustrant par des dessins des exemples de réalisation ,
La figure 1 de la planche 1/5 montre une vue d'ensemble d'un premier type de dispositif de distraction pour les os
La figure 2 de la planche 1/5 est une vue de dessus de l'ensemble du dispositif de distraction (premier mode de réalisation)
Les figures 3 , 4 et 5 de la planche 1/5 montrent trois exemples de moyens d'accrochage aux os.

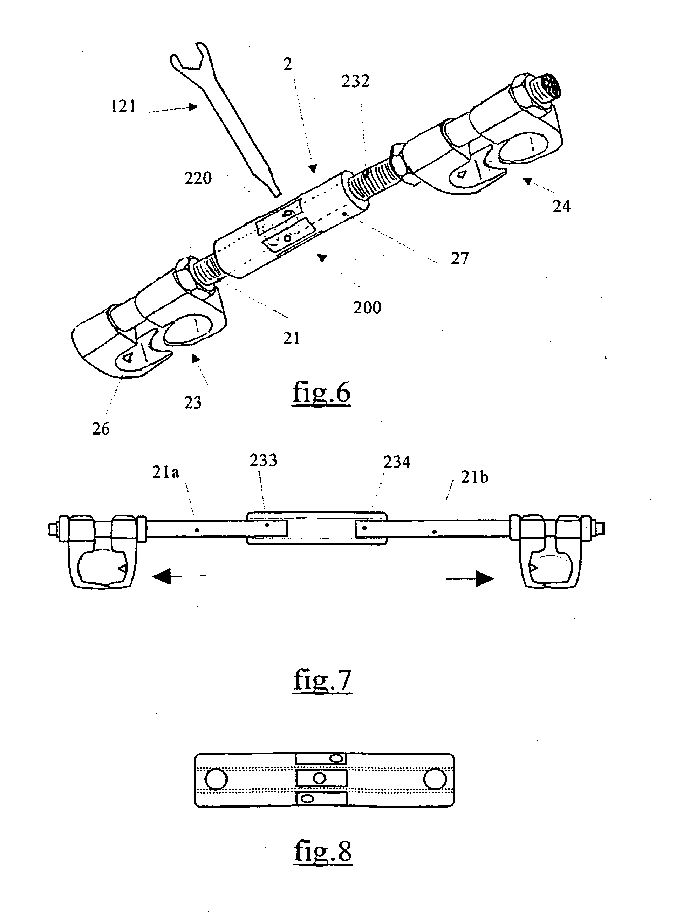

La figure 6 de la planche 2/5 montré une vue d'ensemble d'une variante du dispositif avec son mini-outil (cas du deuxième mode de réalisation)
La figure 7 de la planche 2/5 montre une vue en coupe (cas du deuxième mode de réalisation)
La figure 8 de la planche 2/5 montre le moyen de réglage de l'ensemble du dispositif de distraction. (cas du deuxième mode de réalisation)

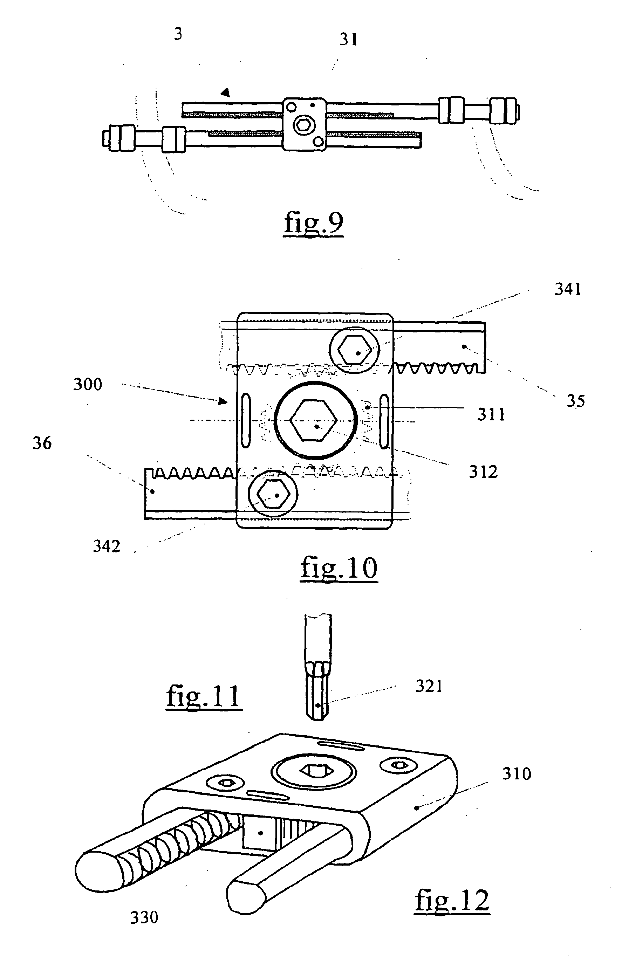

La figure 9 de la planche 3/5 montre une vue de dessus du dispositif de distraction dans le troisième mode de réalisation.
Les figures 10, 11 et 12 de la planche 3/5 montrent les détails du dispositif de réglage du dispositif de distraction en vue de dessus, un exemple de mini-outil de réglage , et une vue en perspective

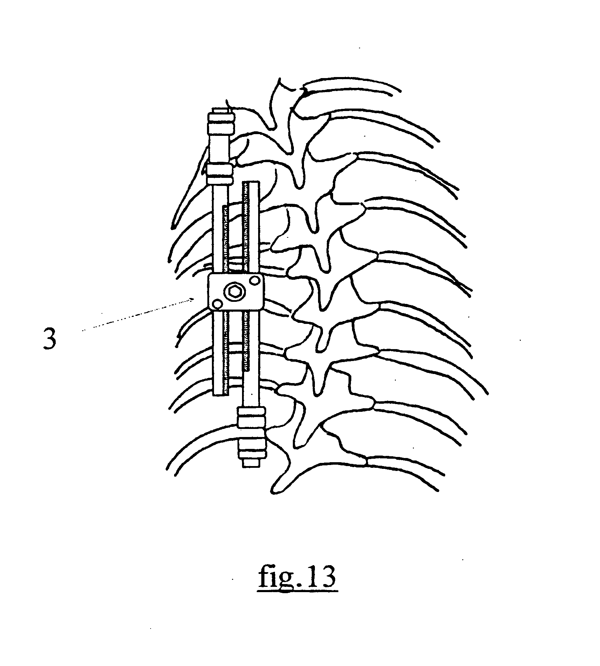

La figure 13 de la planche 4/5 montre un des dispositifs de distraction en place sur l'ossature.
La figure 14 de la planche 5/5 montre une vue en coupe verticale d'un nouveau dispositif de distraction dans le mode de réalisation n°3 comportant aux extrémités des tiges cintrables ou déformables supportant des étriers à leurs extrémités.
Les figures 15 et 15d de la planche 5/5 montrent une vue en perspective d'un accrochage comportant à une extrémité une vis de serrage logée dans un renflement et le détail des mors d'une pince de préhension.

Un premier mode de réalisation est un dispositif de distraction 1 qui comprend : un dispositif de réglage 100 et un dispositif d'accrochage aux os .

Le Dispositif de réglage 100 comprend: au moins une tige filetée 11 avec en son centre un moyen de réglage 12 qui peut être monobloc avec les tiges ou engagé sur ces tiges, permettant le réglage du dispositif de distraction 1. Il possède en son centre un petit trou central 120 d'engagement d'un mini-outil 121,la forme du trou étant adaptée à l'outil, ce qui qui va autoriser un déplacement des moyens du dispositif d'accrochage situés sur les os et de régler ainsi leur écartement.

La tige filetée 11 possède dans ses 2 parties symétriques des pas de vis inversés (gauche et droite) sur lesquels viennent se visser deux dispositifs d'accrochages des os 13 et 14.

Ces dispositifs d'accrochage 13 et 14 peuvent être dans une première version deux flasques 131 et 132 s'accrochant à l'os en épousant parfaitement sa forme , elles sont munies de vis de fixation 133 et 134 déportées par rapport à l'axe du dispositif de réglage.

Lorsque l'enfant grandit , on pratique une petite incision exactement à l'endroit ou se trouve placé le trou 120 correspondant au passage du mini-outil, et on vient régler les dispositifs d'accrochage 13 et 14 , en évitant tous les inconvénients décrits dans le cas des inventions de l'art antérieur qui sont incapables de suivre la croissance.

Dans un deuxième mode réalisation qui est une variante du premier mode de réalisation le dispositif de réglage 200 comprend :
un fourreau central 27 permettant l'écartement recherché entre deux os. Ce fourreau central 27 de réglage possède au moins un mini-trou 220 ou plusieurs décalés pour permettre un accès plus facile par exemple d'une mini-tige que l'on peut rentrer pour régler à l'endroit désiré la distance entre les deux os. Chaque tige filetée à pas inversé rentre dans le moyen central qui comprend de chaque coté un pas de vis adapté et sur sa forme extérieure des méplats ou des pans coupés permettant de le faire tourner par la prise externe d'une mini-clef adaptée à sa forme. Ceci permet au chirurgien d'intervenir pour le réglage du dispositif.

Ce dispositif de réglage 200 s'intégre très facilement dans le corps en protégeant les parties actives 233 ou 234 (des tiges filetées 21a ou 21 b) des tissus et de l'ossature environnante.

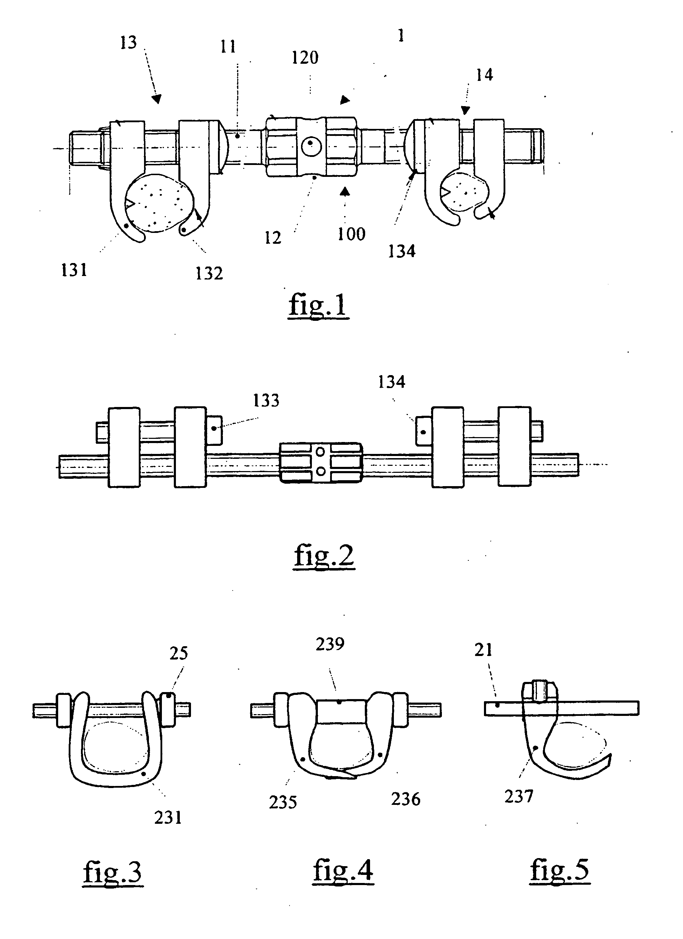

Le dispositif d'accrochage est dans ce cas constitué par exemple de moyens d'accrochages 23 et 24 qui peuvent être :
soit : des étriers 231 qui s'enfilent sur les tiges filetées 21a ou 21b avec blocage par un écrou 25
soit : des crochets 235 et 236 formés de deux parties mâle et femelle épousant la forme de l'os
soit encore un crochet coulissant 237 sur une tige 21 qui peut être lisse , filetée , ou molletée et sur laquelle le crochet vient se fixer par une vis 2370 noyée dans un renflement faisant partie du crochet 237 (figure 5).

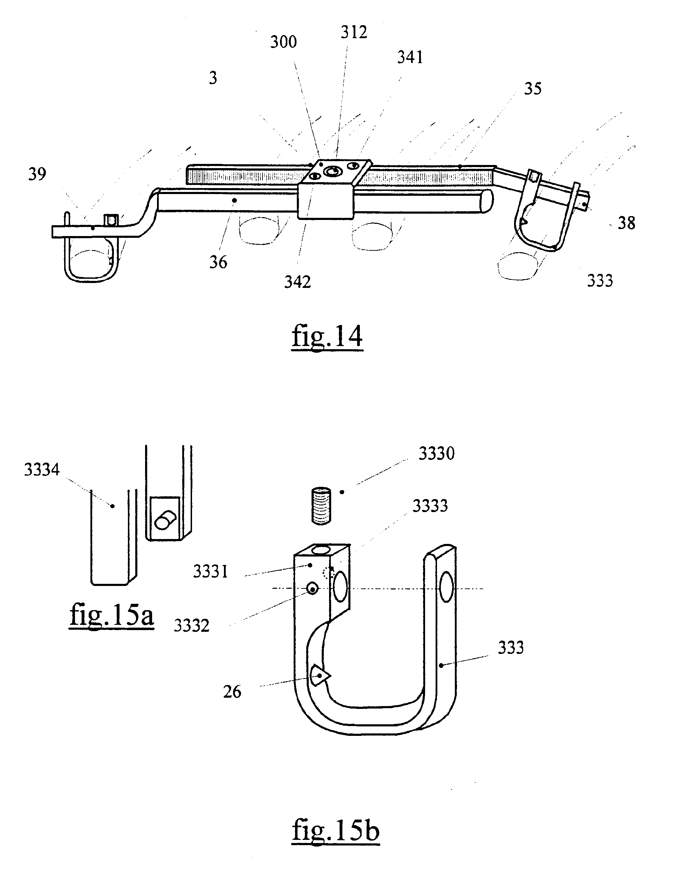

En plus de ces moyens d'accrochage (figure 3,4 et 5), les crochets ou les étriers peuvent posséder une pointe 26 (fig 15) qui vient poinçonner l'os pour mieux l'immobiliser. De plus ,il est préférable de placer une entretoise tubulaire 239 entre les crochets 235 et 236 afin d'éviter tout glissement de l'os par ouverture des crochets au serrage, permettant ainsi d'obtenir un cerclage très efficace de l'os.

Le troisième mode de réalisation du dispositif de distraction 3 se compose :
d'un dispositif de réglage 300 qui comprend un moyen central 31 lui-même composé d'un petit carter 310 (inférieur à la dimension par exemple d'un domino) ,dans lequel tourne librement sur un axe une petite roue qui peut être dentée 311 centrée sur ledit carter 310 et possédant à l'une des extrémités de son axe un moyen 312 de mise en rotation avec l'amplitude désirée .Ce moyen peut être un trou permettent l'engagement d'une clef mâle à six pans 321 ,ou tout autre moyen équivalent, sa seule nécessité est d'être encastré sans proéminence dans ledit carter 310.

Dans ce carter viennent se positionner de chaque coté de la roue 311 deux tiges 35 et 36 qui vont être mises en translation sur demande grâce au moyen de réglage 31 situé sur le carter 310 et accessible par un mini-outil 321. Les deux tiges 35 et 36 peuvent posséder une partie cannelée suivie d'une partie filetée qui permettent d'engager très facilement de manière symétrique les parties filetées jusqu'a la partie cannelée , permettant ensuite l'engrènement systématique dans la roue 311. Avant de procéder au réglage définitif, dés l'engrenement des tiges, on peut engager le mini-outil dans le dispositif de réglage 31 pour amorcer la mise en distraction qui va se faire alors de manière quasi automatique et se poursuivre en utilisant le mini-outil pour parfaire le réglage. Cette opération se fait bien entendu après avoir accroché aux os les moyens d'accrochage précédemment décrits. Dans ce mode de réalisation, le blocage du dispositif de réglage 31 s'opère par serrage de deux vis 341 et 342 situées sur le carter 310. D'autre part un petit cache constitué d'une plaque verticale 330 située entre les deux tiges, ou une plaque ajourée pour les laisser passer, ferme le carter en évitant une introduction notamment de tissus dans le carter , sans perturber en quoi que ce soit le mouvement des tiges 35 et 36 mise en translation par la roue dentée sur action du chirurgien.

Dans le mode de réalisation n°3 le dispositif de distraction 3 va permettre également de corriger une déformation pouvant évoluer dans un espace tridimensionnel.

Pour obtenir un tel résultat on met en place de préférence dans le dispositif de distraction 3
- un nouveau moyen d'accrochage de type étrier 333
- des tiges 35 et 36 aux extrémités cintrables ou déformables 38 et 39.Ce nouvel étrier 333 possède une vis de serrage 3330 logée dans un renflement 3331 faisant partie du corps de l'étrier , ainsi que deux petits trous 3332 et 3333 permettant l'accès à une pince spécifique 3334 dont les mors peuvent se positionner dans les trous pour pouvoir soulever la côte ou l'os et amener la tige 35 ou 36 à s'enfiler sur ledit moyen d'accrochage 333. Cette manipulation est rendue possible par le fait que les extrémités des tiges 38 et 39 sont déformables sur mesure. Une fois le dispositif de distraction 3 mis en place on procède au serrage des vis 3330 on écarte ou on rapproche les accrochages à la distance désirée et on serre le moyen de réglage central 300 par les vis de réglage 341 et 342.Cette manipulation est sans risque pour l'enfant car le cintrage sur mesure des extrémités de tiges 38 et 39 autorise un mouvement de translation pure , rendu impossible avec des tiges droites et rigides sur toute la longueur.

En outre il n'y a aucune erreur possible de manipulation du fait que la vis de réglage 3330 logée dans le renflement 3331 est imperdable ,ce qui permet donc, grâce aux moyens précédemment décrits de corriger une déformation tridimensionnelle sans risque d'erreur.

Grâce aux moyens précédemment décrits dans le mode de réalisation préférentiel n° 3, il est possible de procéder à un montage mécanique sur les os ou les côtes qui se réalise de manière aisée de la façon suivante :
on commence par positionner l'étrier 333 avec sa tige 35 autour de l'os sans le serrer par la vis 3330, ceci autorise une rotation de l'ensemble autour de l'os qui permet ainsi un enfilage des deux tiges 35 et 36 avec leur moyen de réglage 31, que l'on fait coulisser jusqu'aux extrémités 38 et 39.

A l'aide du moyen de réglage 31, on amène la deuxième tige(par exemple 36) dans le moyen d'accrochage 333 prépositionné sur la deuxième cote qui n'est pas forcément la plus proche; cette mise en place n'est réalisable que grâce aux extrémités cintrables sur mesure 38 et 39 des tiges 35 et 36 qui permettent de parvenir à un positionnement dans pratiquement la plupart des cas difficiles rencontrés. Il ne reste plus, une fois les règlages bien déterminés, qu'à effectuer tous les serrage des divers moyens du disposif de distraction 3.
Les trois modes de réalisation du dispositif de distraction 1, 2, 3, permettent donc, grâce aux moyens mis en oeuvre, précédemment décrits de redresser l'ossature d'un enfant, sans pour autant la bloquer définitivement, puisqu'il suffit d'une petite intervention pour accéder facilement au réglage du dispositif de distraction, ce qui permet de suivre les évolutions de cette ossature en corrigeant les déformations tout en permettant à l'enfant de grandir. Les modes de réalisation 2 et 3 les moyens des dispositifs d'accrochages sont parfaitement interchangeables, ils ne sont spécifiques que pour le premier type de réalisation. Le troisième type de réalisation se distingue cependant le plus de l'art antérieur.

Enfin tous ces dispositifs qu'ils soient réalisés dans n'importe quel mode de réalisation comportent des moyens de réglage ou d'accrochage que l'on peut adapter à toutes les formes d'os ; ils devront être fabriqués dans des matériaux biocompatibles avec le corps humain. Comme on le sait actuellement la préférence va vers les métaux inoxydables : acier inoxydable , alliage de titane ou autre métaux de haute résistance, insensibles à la corrosion du corps humain ; les matériaux composites de haute résistance et biologiquement compatibles peuvent être également envisagés. Compte tenu de leur faible dimension, tous ces moyens peuvent être rangés dans des "kit d'assemblage";ces kit étant présentés dans des coffrets bien en ordre pour l'opération. Tous ces nouveaux dispositifs donnent à cette invention une avancée importante dans les dispositifs techniques déjà utilisés pour les soins des malformations qui vont pouvoir être traitées sans interdire la croissance par des dispositifs simples.

## Revendications

1. **Dispositif de distraction (3) permettant de gérer** l'évolution de la déformation du tronc d'un enfant au cours de sa croissance implantable dans le corps humain comportant :
- des moyens d'accrochage aux os (231,235,236,237 et 333)
- un moyen de réglage central (300) pourvu d'un petit trou (312) d'engagement d'un petit outil (321) destiné à régler la distance qui sépare les moyens d'accrochage aux os (231,235,236 ou 239, 333) comprenant un moyen central(31) composé lui-même d'un mini-carter (310) à l'intérieur duquel se trouve une roue dentée (311) centrée sur le dit mini-carter et possédant à l'une des extrémités de son axe un moyen de rotation (312), la roue dentée (311) entraînant en sens inverse deux tiges (35 et 36) positionnées de chaque coté du moyen de réglage (31) par conséquent non situées sur le même axe;
- lesdits moyens d'accrochage étant pourvus de moyens de réglage et de blocage (25 et 3330) se fixant aux extrémités desdites tiges (35) et (36) qui sont des étriers (231) ou (333) qui s'enfilent sur lesdites tiges (35) et (36) ou des crochets (235 et 236) formés de deux parties mâles et femelles épousant la forme de l'os, ou encore un crochet coulissant (237) sur une tige (21) lisse ou filetée ou moletée sur laquelle ledit crochet (237) vient se fixer par une vis noyée (2370) dans un renflement faisant partie dudit crochet;
- des moyens de blocage qui sont des vis (341) et (342) situées sur le mini-carter (310) à proximité du moyen de réglage (300) et qui permettent de bloquer les deux tiges (35) et (36) décalées, dans une position désirée par l'opérateur.

2. Dispositif de distraction (3) selon la revendication 1 **caractérisé en ce que** les deux tiges (35 et 36) sont rectilignes et cintrables sur mesure et composées de deux parties:
- une première partie permettant l'engagement desdites tiges dans le mini carter (310) et de fixer les moyens d'accrochage (231, 235, 236,239)
- une deuxième partie desdites tiges (35) et (36) cannelée permettant un engagement systématique de la roue dentée (311) logée dans le petit carter (310) autorisant ainsi un déplacement opposé des tiges.

3. **Dispositif de distraction(3) selon la revendication 1 caractérisé en ce que** son carter (310) comporte un petit cache (300) constitué d'une plaque entre les deux tiges (35 et 36) ou d'une plaque ajourée pour les laisser glisser tout en fermant ledit carter , en évitant ainsi l'introduction notamment des tissus dans le carter et en laissant libres le mouvement des tiges (35 et 36).

4. Dispositif de distraction(3) selon les précédentes revendications 1,2 et 3 **caractérisé en ce qu'**il comprend :
- au moins un moyen d'accrochage par exemple du type étrier (333)
- et **en ce que** les deux tiges (35 et 36) sont cintrables ou déformables autorisant un maximum de distance en distraction, en translation, sans risque d'agression pour le corps humain.

5. Dispositif de distraction(3) selon la revendication 1 **caractérisé en ce que** son étrier (333)possède des trous de préhension (3332 et 3333) prévus pour une pince spécifique (3334) dont les mors viennent se positionner dans lesdits trous pour soulever les côtes ou les os et ainsi pouvoir les amener à s'enfiler sur les moyens d'accrochage , grâce à la cintrabilité des tiges (35 et 36) notamment de leurs extrémités (38 et 39).

6. Dispositif de distraction (3) selon la revendication 1 **caractérisé en ce que** le moyen d'accrochage (333) possède une vis de serrage logée dans un renflement (3331) imperdable et prépositionnée lors de la mise en place du dispositif (3) et permettant une fois celle-çi effectuée de fixer par serrage de ladite vis les moyens d'accrochage aux extrémités des tiges (38 et 39).

## Claims

1. Distraction device (3) permitting controlling the development of the trunk deformity of a child in growth implantable in human body, including:
- Hooking means to the bones (231,235,236,237 and 333)
- a central adjustment means (300) provided with a small hole (312) for the introduction of a small tool (321) adapted to adjust the distance between the hooking means to the bones (231,235,236 or 239, 333) including a central mean (31) composed of a small frame (310) including itself a teeth wheel (311) centered in the said frame and offering at one of its axis extremity a rotation mean (312), the toothed wheel (311) moving in opposite directions two rods (35 and 36) placed in each side of the adjustment means (31), consequently not on the same axis.
- the said hooking means provided with adjustment and locking means (25 and 3330) fixed to the said rod extemities (35) and (36), that are stirrup (231) or (333) that slip on the said rods (35) and (36) or hooks (235 and 236) composed of one male and one female part surrounding the bone, or a slipping hook (237) fixed to a smooth or threaded rod (21) by an inside screw (2370) in an enlargement of the said hook.
- Blocking means which are screws (341) and (342) placed on the frame (310) near the adjustment mean (300) enabling to block two non aligned rods (36) and (35).

2. Distraction device (3) according to claim 1 **characterized in that** the two rods (35 and 36) are straight and bendable on demand and comprised of two parts:
- a first portion permitting engagement of said rods in a small frame (310) and fixing the hooking means (231,235,236,239,)
- A second portion of said rods (35) and (36) that is channeled, permitting systematic engagement in a toothed wheel (311) disposed in a small frame (310) thereby permitting opposite movement of the rods.

3. Distraction device (3) according to claim 1 **characterized in that** its frame (310) comprises a small cover (300) constitued by a plate between the two rods (35 and 36) or a plate that is pierced to let them slide closing said frame, thereby avoiding the introduction particularly of tissue into the frame, whilst leaving free the movement of said rods.

4. Distraction device (3) according to claims 1,2 and 3 **characterized in that** it comprises:
- at least one hooking means of the stirrup type (333)
- And that theTwo Rods (35 and 36) are bendable or deformable allowing to maximize the distance in distraction in translation, without any risk of aggression to the human body.

5. Distraction device (3) according to claim 1 **characterized in that** its stirrup (333) has holes (3332 and 3333) for gripping, provided for a specific gripper (3334) whose jaws are positionned in said holes to raise the ribs or bones and thus to be able to bring them, thanks to the bendable rods (35 and 36) particularly their ends (38 and 39).

6. Distraction device (3) according to claim 1 **characterized in that** its hooking mean (333) has a tightening screw (3330) disposed in an enlargement (3331) which is hence proof against loss and is pre-positioned during the emplacement of the distraction device (3) and permitting, once this positioning has taken place, fixing, by tightening said screw, the hooking means to the rods ends (38 and 39)

## Patentansprüche

1. Vorrichtung zum Auseinanderziehen (3), die es ermöglicht, die Entwicklung des Oberkörpers eines Kindes im Laufe seines Wachstums zu steuern. Diese Vorrichtung wird in den menschlichen Körper implantiert und hat folgende Eigenschaften:
- Die Befestigungselemente der Knochen (231, 235, 236, 237 und 333)
- Ein Hauptbestandteil des Einstellsystems (300) hat eine kleine Bohrung (312), vorgesehen zum Regulieren der Distanz zwischen den Befestigungselementen und den Knochen (231,235, 236, 237 oder 333) mit einem kleinen Werkzeug (321). Dieser Hauptbestandteil des Einstellsystems (300) besteht aus einem Mittelteil (31), das aus einem Minigehäuse (310) besteht, in dessen Mitte sich ein Zahnrad (311) befindet, zentriert auf dem Minigehäuse, an dessen einem Ende seiner Achse ein Drehelement (312) angebracht ist. Das Zahnrad (311) bewegt in entgegengesetzter Richtung zwei Stäbe (35 und 36), die an jeder Seite des Mittelteils (31) angebracht sind und sich daher nicht auf der gleichen Achse befinden.
- Die Befestigungselemente sind versehen mit Einstell und Blockiervorrichtungen (25 und 3330) und werden an den äußeren Enden der Stäbe (35) und (36) befestigt, diese Vorrichtungen sind entweder Bügel (231 oder 333), die auf die erwähnten Stäbe aufgesetzt werden oder Haken (235 und 236), die aus einem männlichen und einem weiblichen Teil bestehen, die sich der Form des Knochens anpassen, oder auch Gleithaken (237) auf einem glatten oder mit einem Gewinde versehenen oder einem geriffelten Stab (21), an dem der Gleithaken mit einer versenkten Schraube (2370) in einer Vertiefung befestigt wird.
- Die Spannelemente sind Schrauben (341) und (342), die sich auf dem Minigehäuse (310) des Hauptbestandteils des Einstellsystems (300) befinden und mit denen die beiden versetzten Stäbe (36) und (35) von der operierenden Person in der gewünschten Position blockiert werden können.

2. Nach Beschreibung 1 der Vorrichtung zum Auseinanderziehen (3) sind die beiden Stäbe (35 und 36) gerade und je nach Anwendung biegbar und bestehen aus zwei Teilen:
- ein erster Teil ermöglicht es, die genannten Stäbe in das Minigehäuse einzuführen und die Befestigungselemente zu fixieren (231, 235, 236, 239)
- ein zweiter Teil der genannten Stäbe (35) und (36), der gerieft ist, ermöglicht ein systematisches Eingreifen in das Zahnrad (311), das im Minigehäuse (310) platziert ist und so eine Bewegung in entgegengesetzter Richtung ermöglicht.

3. Nach Beschreibung 1 der Vorrichtung zum Auseinanderziehen (3), sitzt im Gehäuse (310) eine Abdeckung (300) zwischen den beiden Stäben (35 und 36), die entweder aus einer Platte besteht oder einer durchlöcherten Platte, durch die die Stäbe hindurchpassen und somit das Gehäuse verschliessen. Dies verhindert ein Eindringen von Gewebe in das Gehäuse und die Stäben (35 und 36) können sich frei bewegen.

4. Nach den Beschreibungen 1, 2 und 3 besteht die Vorrichtung zum Auseinanderziehen (3) aus:
- mindestens einem Befestigungselement des Typs Bügel (333)
- und zwei biegbaren und verformbaren Stäben (35 und 36), die eine maximale Parallelverschiebung ermöglichen, ohne dem menschlichen Körper zu schaden.

5. Nach der Beschreibung 1 der Vorrichtung zum Auseinanderziehen (3) besitzt der Bügel Greiflöcher (3332 und 3333), die für eine bestimmte Zange (3334) vorgesehen sind, deren Backen in diese erwähnten Greiflöcher gesetzt werden, um die Rippen oder die Knochen anzuheben. Die Rippen oder Knochen können so dank der biegbaren Stäbe (35 und 36), insbesondere deren Enden (38 und 39), auf die Befestigungselemente aufgesetzt werden.

6. Nach der Beschreibung 1 der Vorrichtung zum Auseinanderziehen (3) besitzt das Befestigungselement (333) eine gesicherte Schraube zum Festziehen, die in einer Vertiefung (3331) sitzt und bereits beim Einsetzen in der Vorrichtung (3) eingebaut ist. Die Befestigungselemente können somit nach dem Einsetzen der Vorrichtung (3) durch die genannte Schraube an den Enden der Stäbe (38 und 39) festgezogen werden.
